# EUROPEAN PATENT SPECIFICATION

(11) **EP 0 242 792 B1**
(45) Date of publication and mention of the grant of the patent: **22.07.1992**
(21) Application number: 87105607.3
(22) Date of filing: 15.04.1987
(51) Int. Cl.: A61K 7/06, A61K 7/08, A61K 7/075

(54) **Hair cosmetic composition**
Haarkosmetisches Mittel
Composition cosmétique pour cheveux

(30) Priority: 25.04.1986 JP 96206/86; 28.04.1986 JP 99193/86
(43) Date of publication of application: 28.10.1987
(73) Proprietor: Kao Corporation, Chuo-ku Tokyo (JP)
(72) Inventor: Noboru, Suzuki, Koto-ku Tokyo (JP); Hajime Hirota, Meguro-ku Tokyo (JP); Kazuyuki, Yahagi, Koto-ku Tokyo (JP)
(74) Representative: Wächtershäuser, Günter, Prof. Dr.

(56) References cited:
- EP-A- 0 130 609
- EP-A- 0 181 547
- GB-A- 2 091 102
- US-A- 3 728 447
- US-A- 3 892 669

## Description

### BACKGROUND OF THE INVENTION

### 1) Field of the Invention

This invention relates to novel hair cosmetic compositions and more particularly to various hair cosmetic compositions, such as hair rinse, hair conditioner, hair treatment, hair cream, styling lotion styling mousse, conditioning mousse, hair spray and the like, which comprise branched alkyl quaternary ammonium salts and a specific type of oily compound and which are rarely felt as oily and have good smoothness, good flexibility and a good antistatic effect.

### 2) Description of the Prior Art

Hitherto, there have been used hair cosmetic compositions, such as hair rinses, which comprise as its main component linear long-chain dialkyl quaternary ammonium salts in which the two long-chain alkyl groups are of the linear type, e.g. distearyl dimethylammonium chloride.

The hair rinses have the purpose of imparting to the hair flexibility, smoothness and antistaticity. However, the use of the linear long-chain dialkyl quaternary ammonium salt alone is not satisfactory with respect to the impartment of the flexibility and smoothness. In order to overcome the above drawback, it is usual to add oils such as higher alcohols, glycerides, liquid paraffin and the like.

In hair creams or styling lotions using oils or resins as a main component, linear, long chain mono or di-alkyl quaternary ammonium salts are employed in order to impart flexibility, smoothness and antistaticity.

Linear, long-chain dialkyl quaternary ammonium salts have not the capability of stably emulsifying or dispersing oils or fats in amounts sufficient to show the effect. To obtain a stable emulsion or dispersion such as for creams or rinses, attempts have been made to formulate highly hydrophilic linear long-chain monoalkyl quaternary ammonium salts or nonionic surface active agents. However, these highly hydrophilic compounds considerably lower the rising effect with the attendant disadvantage that the rinsing effect inherent to the quaternary ammonium salts and oils or fats cannot be shown satisfactorily.

With hair cosmetic compositions comprising oils or resins as a main component, known linear long-chain mono or di-alkyl quaternary ammonium salts cannot show the effect of imparting good flexibility, smoothness and antistaticity.

Accordingly, there is a demand of hair cosmetic compositions which are excellent with respect to flexibility, smooth and antistaticity and are not felt as oily even though large amounts of resins and oils or fats are present.

### SUMMARY OF THE INVENTION

Under these circumstances in this art, the present inventors made intensive studies. As a result, it was found that when part or a whole of known linear, long-chain alkyl quaternary ammonium salts are substituted with a specific type of branched quaternary ammonium salt and are used in combination with a specific type of oily compound, the resulting hair cosmetic compositions can overcome the above prior art problems and ensure a high degree of set retention under high humidity conditions. The present invention was accomplished based on the above finding.

The present invention provides hair cosmetic compositions which comprise the following two ingredients (A) and (B):
(A) a total of 0.01 to 20 wt% of one or more of the following branched quaternary ammonium salts (i) or (ii): 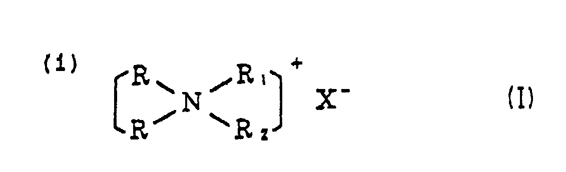 in which each R represents an alkyl group selected from the group consisting of (a) branched alkyl group of the formula 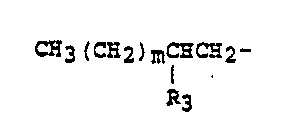 and (b) a linear alkyl group of the formula 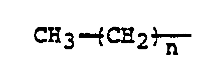 (in which R₃ represents a methyl or ethyl group, and m and n are independently a value determined such that the total carbon atoms in the alkyl groups range from 8 to 16), and the branching ratio (a)/(a)+(b) is in the range of 10 to 100 wt%, R₁ and R₂ are independently a group selected from the group consisting of a benzyl group, and an alkyl group and a hydroxylalkyl group each having 1 to 3 carbon atoms, and X⁻ represents a halogen ion or an organic anion, and 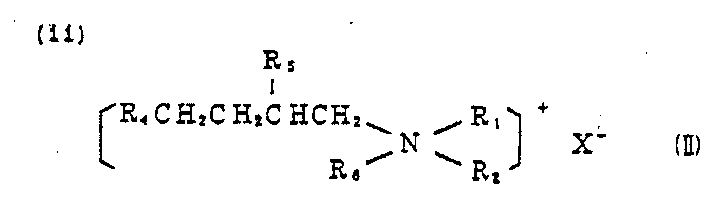 in which R₄ and R₅ independently represent an alkyl group having 2 to 16 carbon atoms, R₆ represents 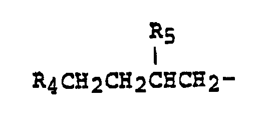 or an alkyl group having 1 to 3 carbon atoms, and R₁, R₂ and X⁻ have, respectively, the same meanings as in formula (I); and
(B) the following oily compound (iii) or (iv):
   (iii) 0.1 to 5 wt% of a long-chain acyl compound of the following general formula (III) or its salt 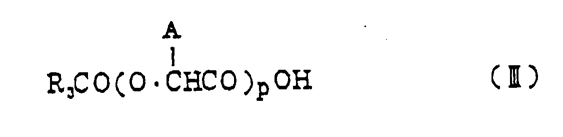 in which R₃CO represents an aliphatic linear or branched acyl group having 6 to 22 carbon atoms, A represents CH₃ or hydrogen, and p is an integer of 1 to 3; and
   (iv) 0.1 to 30 wt% of α-mono(methyl branched alkyl)glyceryl ether represented by the following general formula (IV)

      R'-OCH₂CH(OH)CH₂OH (IV)

      in which R' represents a methyl branched alkyl group having 12 to 24 carbon atoms.

### DETAILED DESCRIPTION OF THE INVENTION AND PREFERRED EMBODIMENTS

Of the ingredients (A) used in the present invention, the branched quaternary ammonium salts (i) are prepared from starting oxoalcohols having 8 to 16 carbon atoms. Examples of the ammonium salts (i) include those having alkyl groups from the oxoalcohols, e.g. dialkyldimethylammonium salts, dialkyl methylhydroxyethylammonium salts, dialkylmethyl-benzylammonium salts, and the like. The counter ions for these ammonium salts include halogen ions such as chlorine, iodine and bromine ions, and organic ions such as methosulfate, ethosulfate, methophosphate, ethophosphate and the like.

Each alkyl group, R, in formula (I) is a group having from 8 to 16 carbon atoms in total and selected from 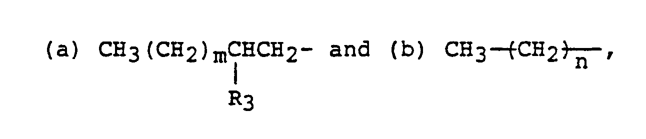
and R₃ is a methyl or ethyl group. The branching ratio of (a)/(a)+(b) in R is obtained from a branching ratio of a starting oxoalcohol and is generally from 10 to 100 wt%, preferably from 10 to 50 wt%. Although the total number of carbon atoms in the alkyl group, R, may be from 8 to 16, preferable alkyl groups should have a given distribution of carbon atoms. Most preferably, those having the following distribution are used.

| | |
|---|---|
| C₈₋₁₁: | below 5 wt% |
| C₁₂: | 10 - 35 wt% |
| C₁₃: | 15 - 40 wt% |
| C₁₄: | 20 - 45 wt% |
| C₁₅: | 5 - 30 wt% |
| C₁₆: | below 5 wt% |

Preferable examples include dialkyldimethylammonium chlorides of the formula (I) whose alkyl groups, R, each has 8 to 16 carbon atoms and a branching ratio of 10 to 50 wt%. When used as a hair rinse, this compound ensures good smoothness of the hair in a wet state.

The branched quaternary ammonium salts (ii) used as the ingredient (A) are those of the formula (II) and are usually prepared from starting Guerbet alcohols of the following formula having 8 to 36 carbon atoms, 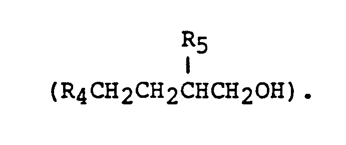
Preferable examples of the branched quaternary ammonium salts are those having an alkyl group derived from the Guerbet alcohol, including mono long-chain alkyl quaternary ammonium salts such as alkyltrimethylammonium salts, alkyldimethylhydroxyethylammonium salts, alkyldimethylbenzylammonium salts and the like; and dialkyldimethylammonium salts, dialkylmethylhydroxyethylammonium salts, dialkylmethylbenzylammonium salts, and the like. The counter ions of these ammonium salts may be halogen ions such as chlorine, iodine and bromine ions, and organic anions such as methosulfate, ethosulfate, methophosphate, and ethophosphate. Examples of the alkyl group derived from the Guerbet alcohol include 2-hexyldecyl, 2-octyldodecyl, 2-decyltetradecyl, and 2-dodecylhexadecyl. Preferable examples of the branched quaternary ammonium salts (ii) include 2-decyltetradecyltrimethylammonium chloride, 2-dodecylhexadecyltrimethylammonium chloride, di-2-hexyldecyldimethylammonium chloride, and di-2-octyldodecylammonium chloride. When formulated in hair rinses, these compounds ensure good smoothness of the hair in a dry state.

The quaternary ammonium salts (i) or (ii) may be used singly or in combination and are used in a total amount of 0.01 to 20 wt%, preferably 0.05 to 5 wt%, of the hair cosmetic composition.

The cosmetic compositions of the invention may further comprise, aside from the above quaternary ammonium salt, cetyltrimethylammonium chloride or stearyltrimethylammonium chloride as an auxiliary.

The oily compound (iii) of the ingredient (B) of the invention is an aliphatic acid lactylate or an aliphatic acid glycollate of formula (III). The compounds of formula (III) in which R₃CO has 12 to 18 carbon atoms are preferred, including lauroyl lactylate, isolauroyl lactylate, myristoyl lactylate, isomyristoyl lactylate, oleloyl lactylate, isooleloyl lactylate, stearoyl lactylate, isostearoyl lactylate, lauroyl glycollate, isolauroyl glycollate, myristoyl glycollate, isomyristoyl glycollate, oleloyl glycollate, isooleloyl glycollate, stearoyl glycollate, isostearoyl glycollate, and the like. The salts may be ammonium salts, alkali metal salts, or amine salts. The long-chain acyl compound of the general formula (III) is incorporated in an amount of 0.1 to 5 wt%, preferably 0.1 to 3 wt%, of the total composition. Outside the range, the effect of the invention cannot be shown satisfactorily.

The oily compound (iv) of the ingredient (B) of the invention is α-mono(methyl branched alkyl)glyceryl ether represented by the formula (IV), which may be synthesized according to the process described in Japanese Patent Publication No. 36260/1982. Preferable oily compounds are those having the following group (V) as R' appeared in the formula (IV): 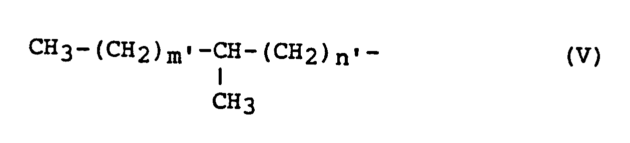
in which m' is an integer of 2 to 14, n' is an integer of 3 to 11, and the sum of m' + n' is 9 to 21. Of these, compounds whose total number of m' and n' in the formula (V) is 11 to 17, that is, a total number of carbon atoms in the alkyl group are 14 to 20, are preferred. Further, the most preferred ones are those having a total number of m' and n' of 15, that is, a total number of carbon atoms in the alkyl group are 18. It is also preferred that a branched methyl group is present near the center of main alkyl chain.

Generally speaking, stock alcohol industrially produced is a mixture of compounds having a specific distribution in respect of total number of carbon atoms in alkyl groups and of position of their branched methyl group. For instance, isostearyl alcohol, a reduced product of isostearic acid which has a branched methyl group and is by-produced during a production of oleic acid dimer, includes compounds having 18 carbon atoms in total (the sum of m' and n' is 15) and compounds having 14, 16, 20 carbon atoms, respectively; an amount of the former is more than 75% of the total alcohol and its branched methyl group is present near the center of the main alkyl chain (J. Amer. Oil Chem. Soc., 51, 522(1974)).

The following data show properties of a typical compound of α-mono(methyl branched alkyl)glyceryl ether.

| Methyl Branched Alkyl Group | Melting Point | Specific Gravity (at 30°C) | Viscosity (at 30°C) |
|---|---|---|---|
| Methyl branched stearyl (mainly m'=7 and n'=8 in the group (V)) | 23°C | 0.912 | 856 |

The α-mono(methyl branched alkyl)glyceryl ether represented by the formula (IV) is incorporatd in the hair cosmetic compositions of the present invention in amounts of 0.1 to 30 wt%, preferably 0.3 to 10 wt%.

The hair cosmetic compositions can be prepared in an ordinary manner which comprises, for example, adding the ingredients (A) and (B) to hot water, and cooling the solution while agitating. For the preparation, solvents such as alcohols, propylene glycol, glycerine and the like may be used. In order to adjust a pH of a 5% aqueous solution to 3 - 8 like ordinary hair rinses, there may be added, if necessary, organic acids such as citric acid, lactic acid and the like, inorganic acids such as phosphoric acid, hydrochloric acid and the like, inorganic alkalis such as caustic soda, and organic alkalis such as triethanolamine.

Aside from the above ingredients, the hair cosmetic compositions of the invention may further comprise, if necessary, liquid paraffin, glycerides, higher alcohols, lanoline derivatives, esters, higher fatty acids, and the like oils. Of these oils, preferable ones suitable for hair rinses or hair conditioners include saturated or unsaturated, linear or branched fatty acid-derived monoglycerides having 12 to 24 carbon atoms, and higher alcohols having a linear or branched alkyl group or alkenyl group having 12 to 26 carbon atoms. Preferable examples include fatty acid monoglycerides such as oleic monoglyceride, palmitic monoglyceride, stearic monoglyceride, behenic monoglyceride and the like, and higher alcohols such as cetyl alcohol, stearyl alcohol, arachidic alcohol, behenyl alcohol, caranabyl alcohol, ceryl alcohol and the like.

If necessary, the hair cosmetic compositions of the invention may further comprise medical agents such as antidundruff agents, vitamines and the like, preservatives such as paraben, thickeners such as water-soluble polymers, colorants such as dyes and pigments, conditioners such as cationic polymers, pearling agents such as glycol esters, and other compounded perfumes.

Preferable formulations of the hair cosmetic compositions containing arbitrary ingredients according to the invention are shown in the Table below.

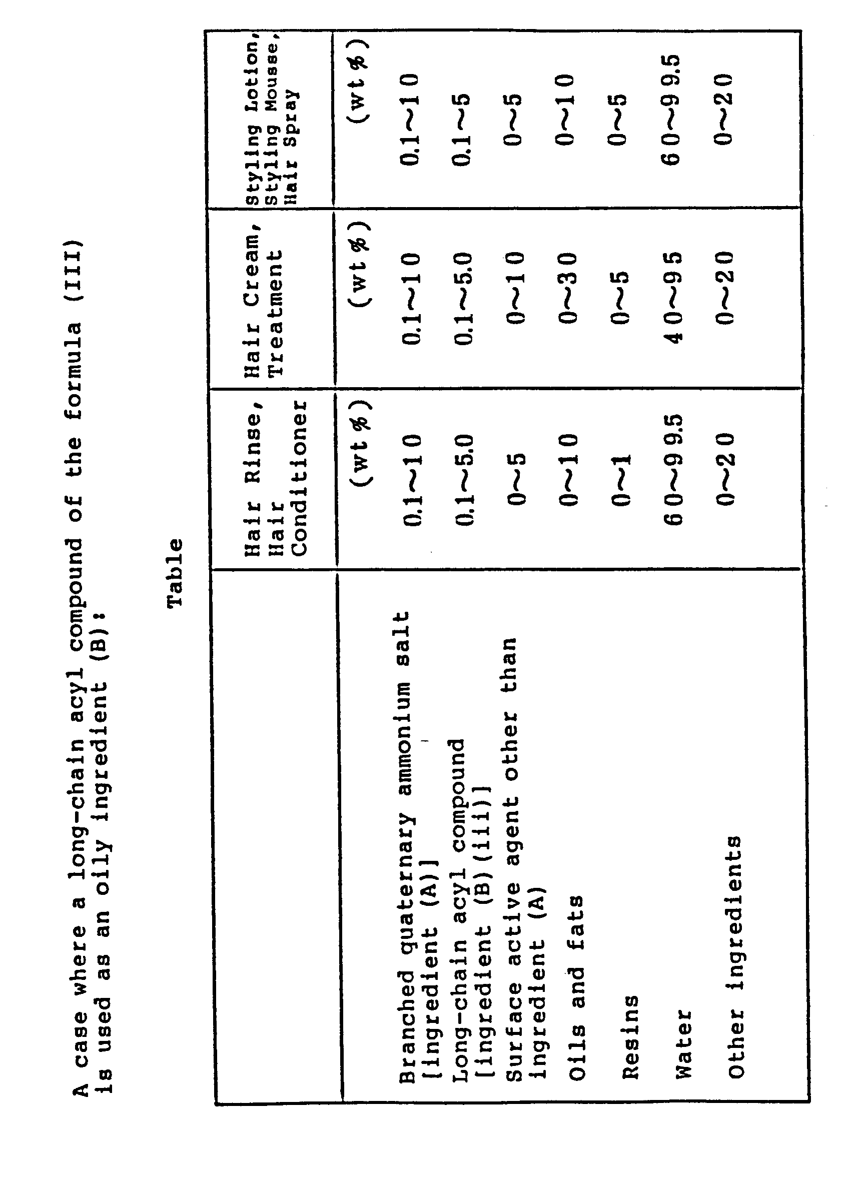

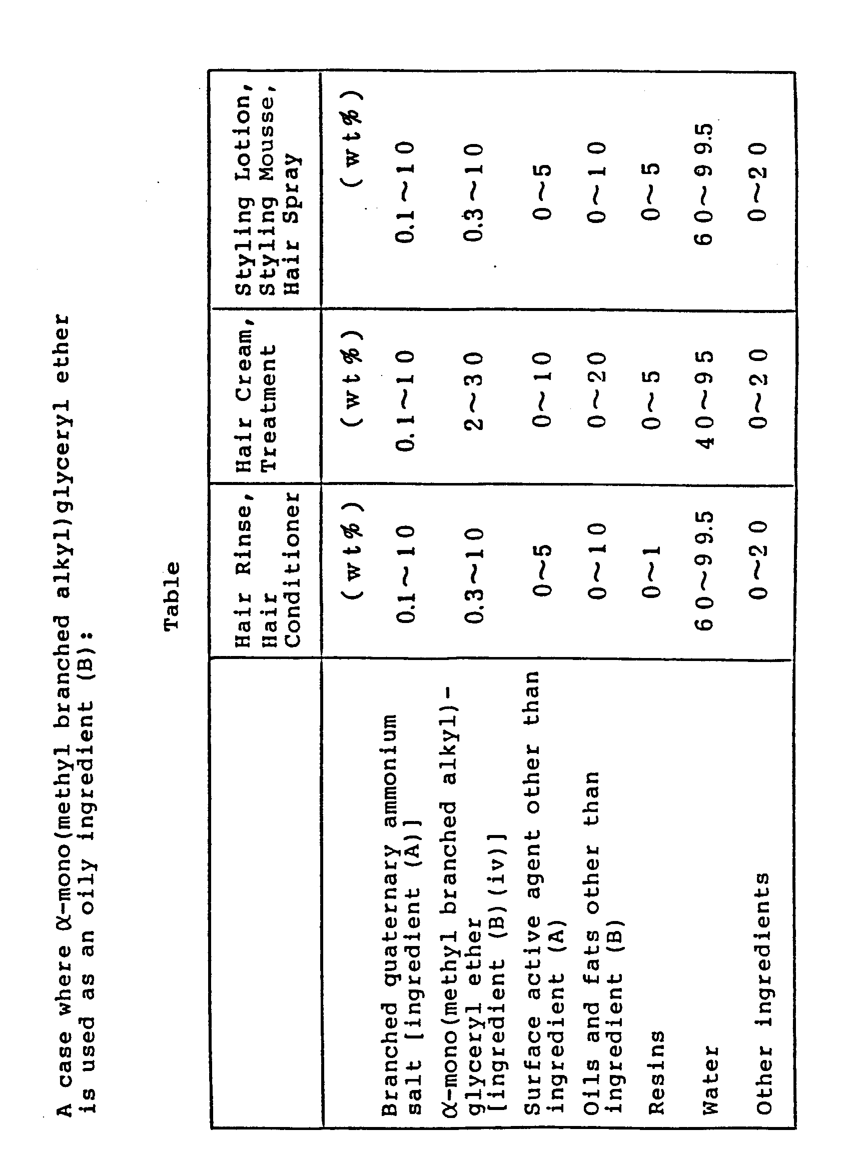

In the case where the hair cosmetic composition of the present invention is formulated into aerosols such as mousse, hair spray and the like, it is desirable to use a propellant such as a fluorocarbon, a liquefied petroleum gas, dimethyl ether or the like, in addition to the above formulations (stock solution), to such an extent that an inner pressure reaches 2.0 - 6.0 kg/cm²G or in an amount of 1 - 20 wt% of the total composition.

The hair cosmetic composition of the invention shows high emulsion and dispersion stabilities after a long period of storage under a low temperature. Further, the composition rarely imposes oily feel and ensures good smoothness, flexibility and antistaticity with a set retention being high under high humidity conditions.

The present invention is described by way of examples, which should not be construed as limiting the invention. The test methods used in the examples are as follows.

### (1) Set Retention

20 g of the hair (15 cm in length) of Japanese females who have never undergone any beauty treatments such as cold perm, bleaching and the like was tied up into a tuft. The hair tuft was applied unifomly with 2 g of a hair rinse, followed by rinsing with running water for 30 second and dried with a towel. This wet hair tuft was wound about a cylinder having a diameter of 15 mm and dried. After drying, the cylinder was gently removed and the hair tuft was allowed to stand under given humidity conditions (65% R.H. and 95% R.H.) for 30 minutes and a degree of variation from the original state was checked. The evaluation was made as follows: "excellent" was indicated as ⓞ, "good" as ○, "moderate" as △, and "poor" as X.

### (2) Rinsing Property (Organoleptic Evaluation)

20 g of the hair (15 cm in length) of Japanese females who have never undergone any beauty treatments such as cold perm, bleaching and the like was tied up into a tuft. The hair tuft was applied uniformly with 2 g of a hair rinse, followed by rinsing with running water for 30 seconds and dried with towel. This wet hair tuft was subjected to organoleptic evaluation with respect to the rinsing property (softness and oiliness) in which "excellent" was indicated as ⓞ, "good" as ○, "moderate" as △, and "poor" as X.

### (3) Stability upon Storage

Each composition was placed in a transparent glass vessel having a capacity of 100 ml as a sample and then stored in a thermostatic chamber at a temperature of 5°C, 20°C, and 40°C, respectively, for one month. After the storage, appearance of each sample was macroscopically observed to evaluate the stability upon storage. Evaluation criteria are as follows.
- ○ :: homogeneous; neither precipitation nor coagulation was observed; high stability upon storage
- △ :: homogeneous, but pearl like crystals or the like were precipitated; less stable upon storage
- X :: nonhomogeneous; precipitation, coagulation and the like were observed; low stability upon storage

### (4) Combing Force

Wet hair tuft (water content was about 0.7 g/g hair) was dried in the air or by using a hair dryer for five minutes. After that, the hair tuft (water content was about 0.1 g/g hair) was set to a strain gauge to measure the combing force. The measurement was carried in a thermo-hygrostatic chamber at 20°C and 65% R.H., and was repeated 20 times. The combing force (g) was determined by taking an average of these 20 values.

### (5) Quantity of Produced Static Electricity

Dried tuft of the hair above obtained was combed 10 times in a thermo-hygrostatic chamber at 20°C and 65% R.H. The quantity of static electricity (kv) produced on the hair was measured.

### (6) Friction Coefficient of the Surface of the Hair

A hair sample of 20 cm long and about 50 µm thick was shampooed twice, followed by a uniform application of 0.5 g of a hair rinse composition on the hair. In a thermo-hygrostatic chamber at 20°C and 65% R.H., a single string of the thus-treated hair was placed on a nylon pulley and a weight of 0.5 g was hung at each end of the hair string. The hair string and the pulley were washed with about 2 g of water. The pulley was then rotated at 5 rpm, and the tension difference between the two ends of the hair string was detected by a strain gauge. Based on a value upon an elapsed time of a predetermined period after initiation of the rotation, a dynamic friction coefficient of the hair was determined. Ten or more hair strings were thus measured with respect to each liquid sample and their friction coefficient data were averaged.

### Example 1

Compositions having the formulations shown in Tables 1 and 2 were prepared to evaluate the rinsing properties and the set retention.

### Preparation of the Compositions:

The ingredients (1) and (3) or (2) and (3) melted by heating to 70°C were added to water (4) heated to the same temperature as indicated above, and agitated for emulsification, followed by cooling to room temperature while agitating, thereby obtaining hair rinse compositions.

### Results:

Shown in Tables 1 and 2.

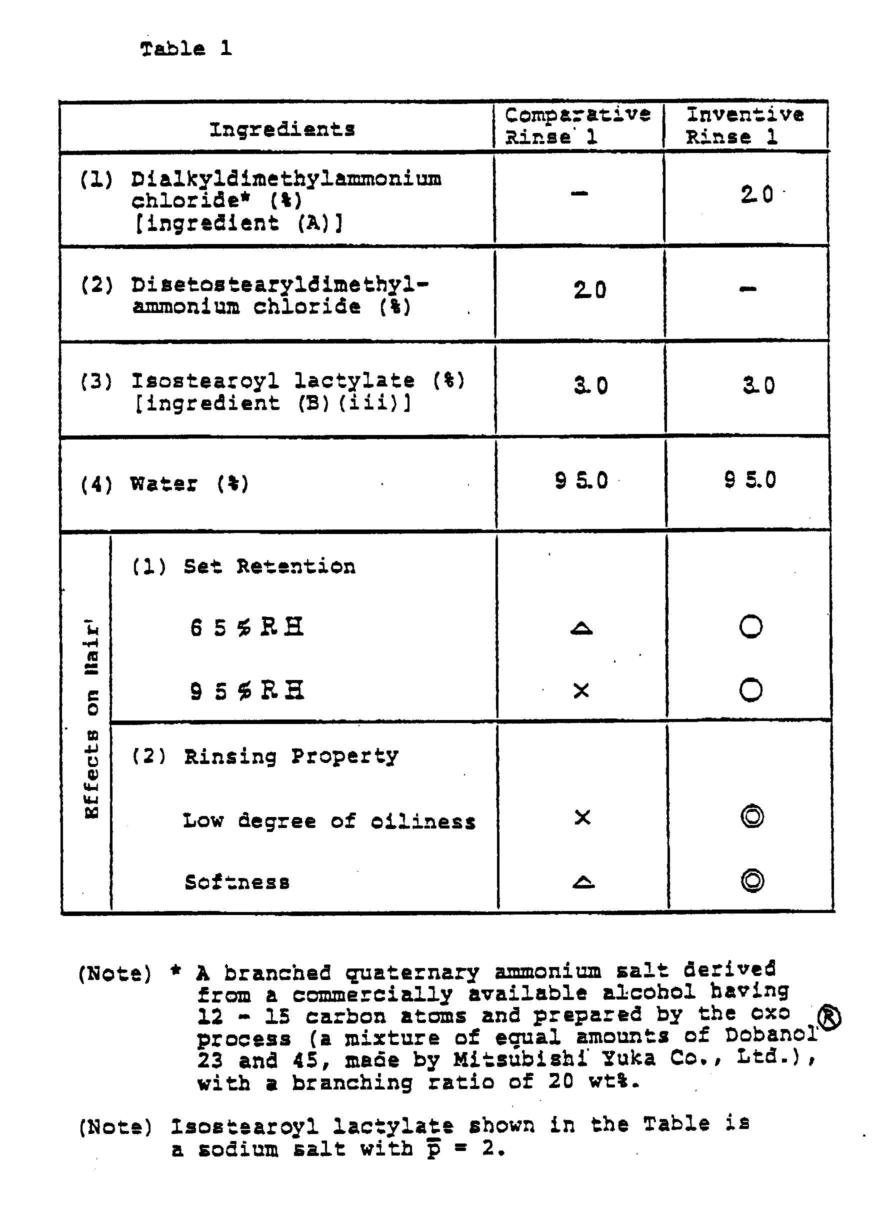

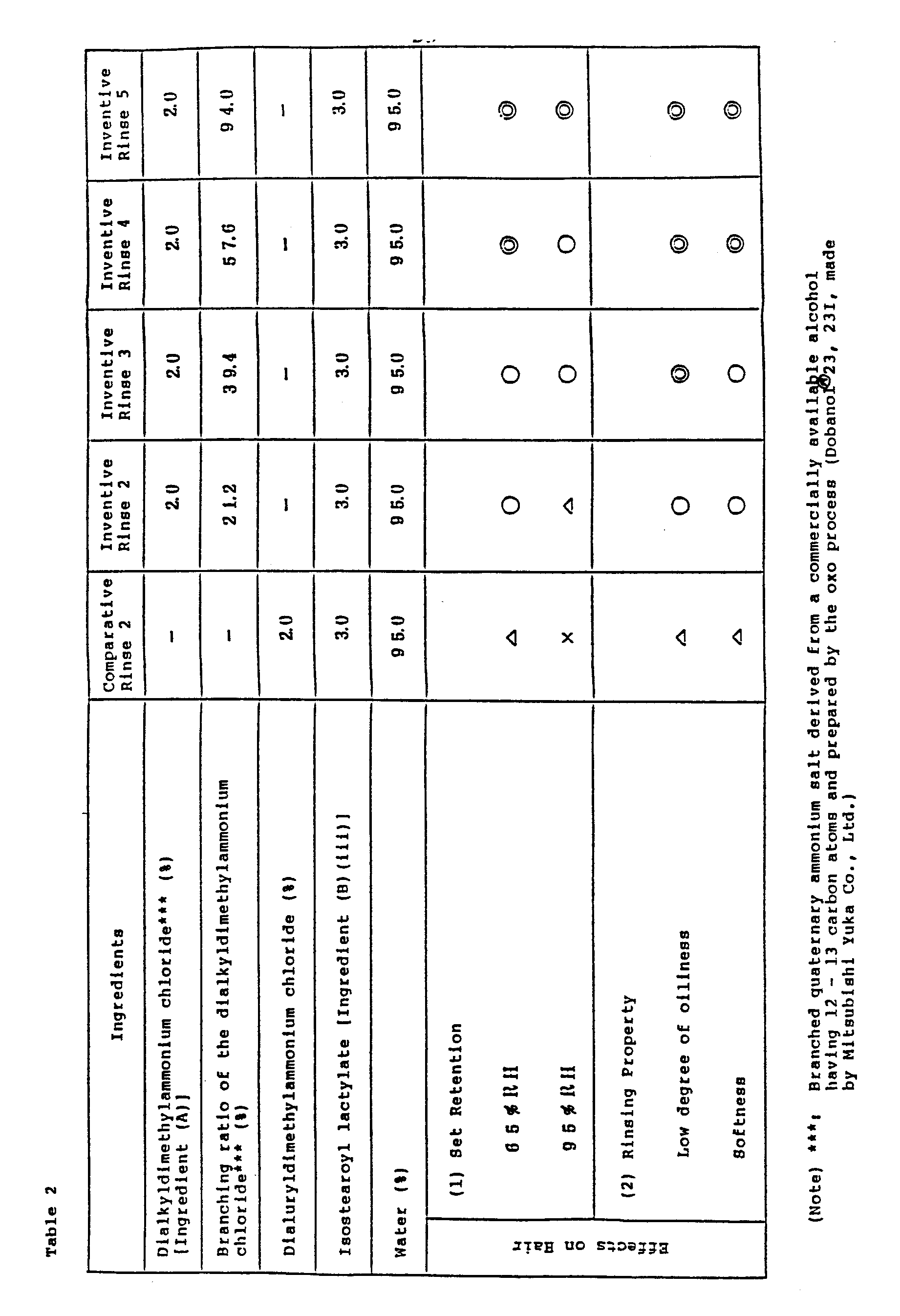

As will be apparent from the Tables 1 and 2, when the ingredients (A) and (B) are used in combination, there can be obtained hair cosmetic compositions which have good rinsing properties and a good set retention under high humidity conditions.

### Example 2

Compositions having the formulations shown in Table 3 were prepared in the same manner as in Example 1 to evaluate the rinsing property and set retention. The results are shown in Table 3.

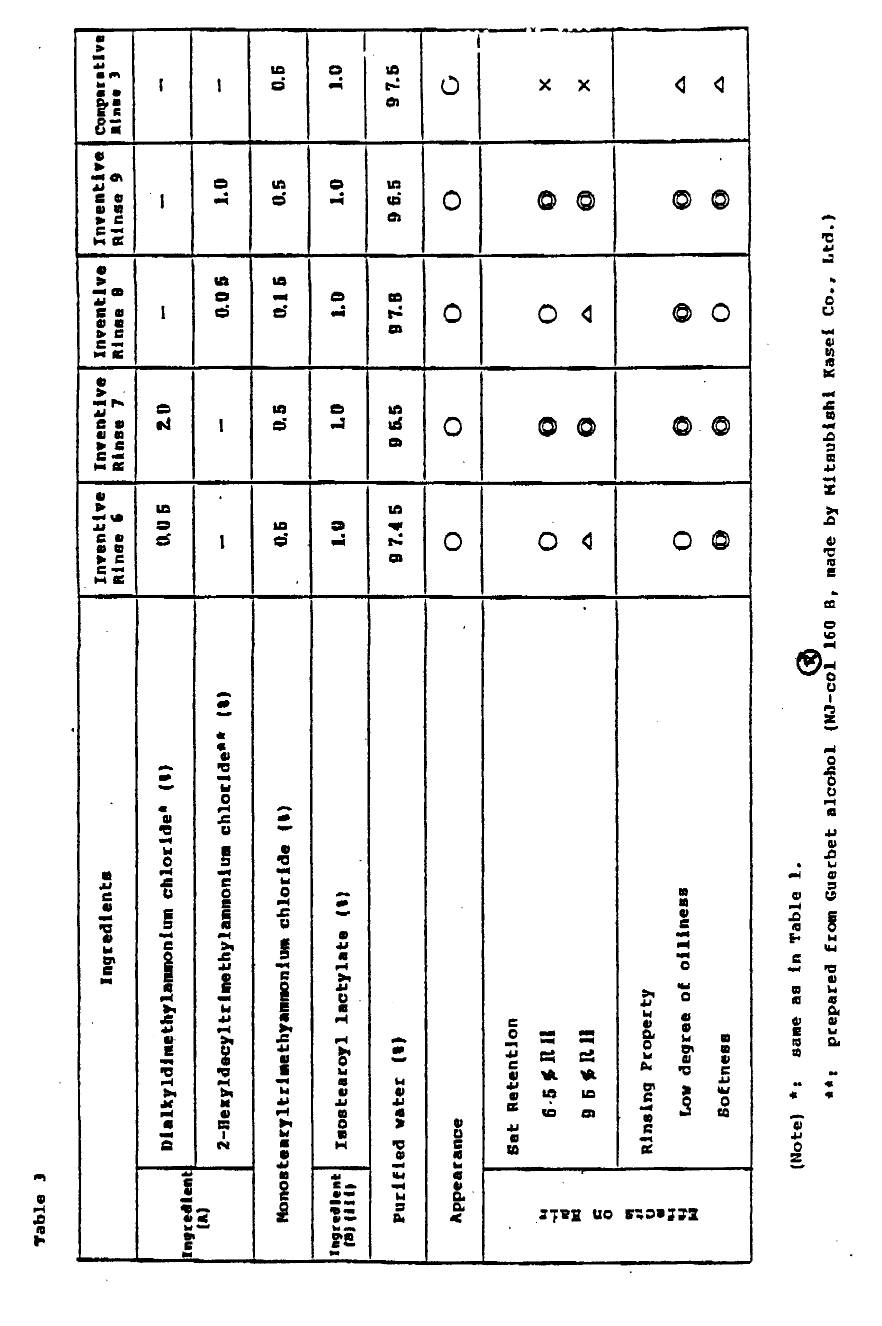

### Example 3

A hair rinse composition of the following formulation was prepared and its rinse property was evaluated by 19 female panelists using a paired comparison method. The comparison standards were determined as +2 when it was very good and as +1 when it was good. The results are shown in Table 4.

### Inventive Rinse 10:

| | |
|---|---|
| (1) Dialkyldimethylammonium chloride^{*} | 2.0(%) |
| (2) Isostearoyl lactylate | 1.0 |
| (3) Propylene glycol | 5.0 |
| (4) Water | 91.6 |
| (5) Perfume | 0.4 |

To (4) heated to 70°C was added a mixture of (1) - (3) which had been melted by heating at the same temperature as indicated above, which was agitated for emulsification, followed by cooling down to 45°C while agitating and further adding (5). The mixture was cooled down to room temperature under agitation to obtain a hair rinse composition. The mark "*" has the same meaning as set forth in Table 1.

### Comparative Rinse 4:

| | |
|---|---|
| (1) Dicetyldimethylammonium chloride | 2.0(%) |
| (2) Isostearoyl lactylate | 1.0 |
| (3) Propylene glycol | 5.0 |
| (4) Water | 91.6 |
| (5) Perfume | 0.4 |

To (4) heated to 70°C was added a mixture of (1) - (3) which had been melted by heating at the same temperature as indicated above and agitated for emulsification, which was cooled down to 45°C under agitation, followed by addition of (5) and cooling down to room temperature under agitation to obtain a hair rinse composition.

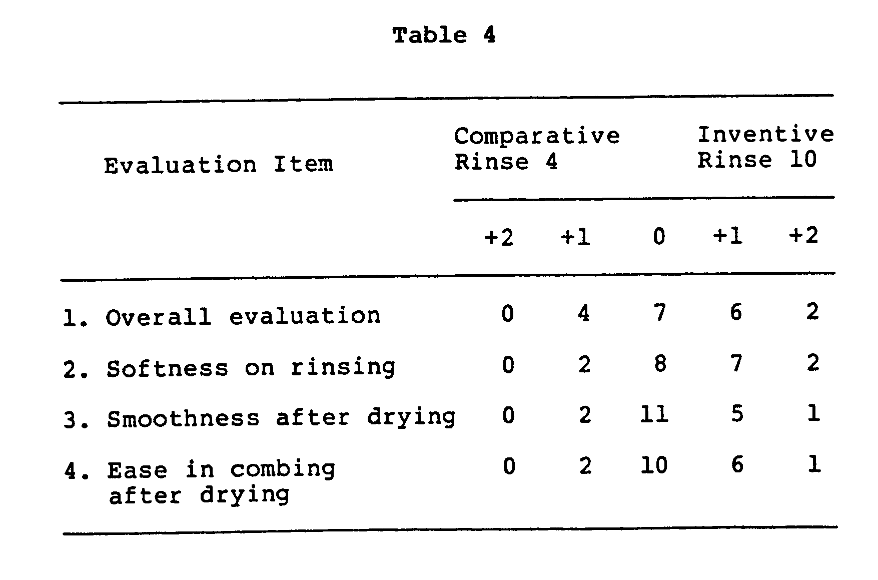

### Example 4

To a mixture of (6), (7), (8) and (10), shown in Table 5, heated to 70°C was added a mixture of (1) - (5) which had been melted by heating at the same temperature as indicated above, which was agitated for emulsification and cooled down to 45°C while agitating, to which (9) was added. The mixture was further cooled down to room temperature under agitation to obtain a hair rinse composition. The mark "*" has the same meaning as set forth in Table 1.

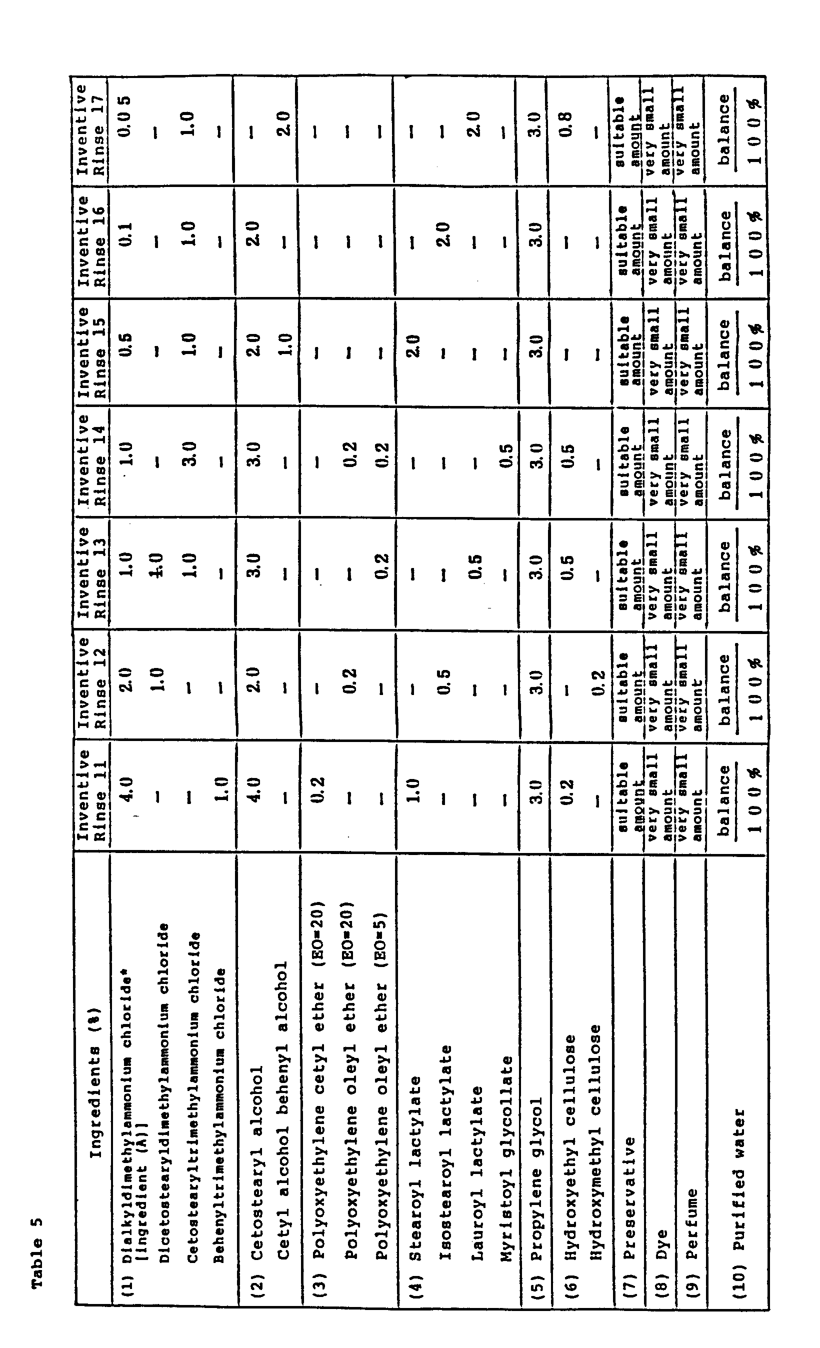

### Example 5

Hair rinse compositions were prepared in the same manner as in Example 1 to evaluate their properties.

The resultant hair rinse compositions had all good rinsing properties and a good set retention.

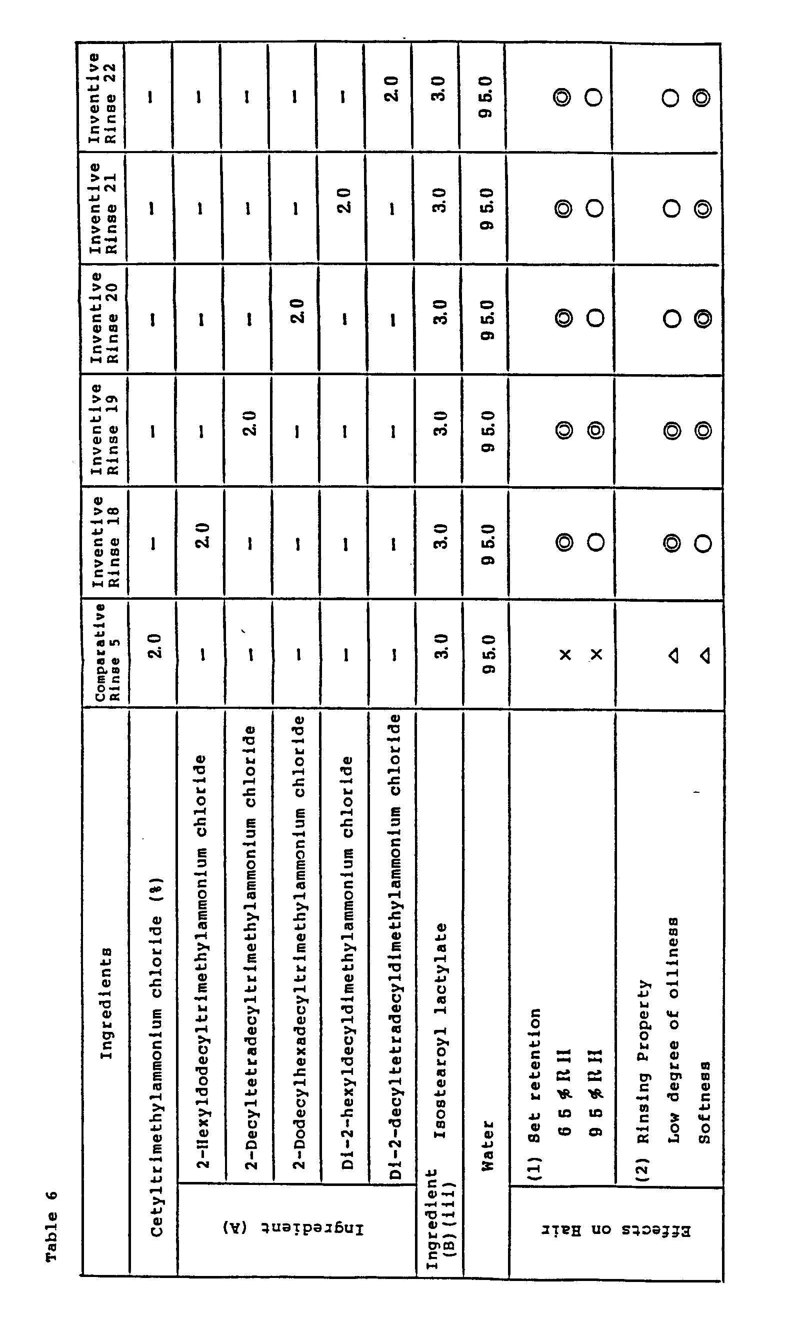

### Example 6

### Hair treatment composition:

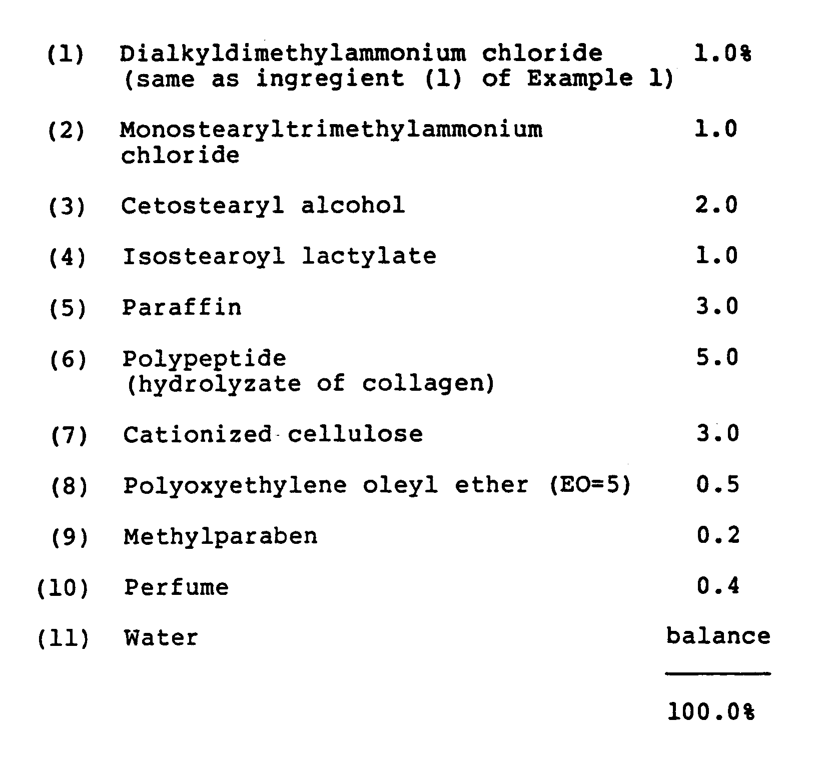

### (Preparation)

(6), (7) and (9) were uniformly dispersed in (11) and heated. A uniform solution of (1), (2), (3), (4), (5) and (8) which had been heated under agitation was added to the dispersion and cooled, to which (10) was added to obtain a hair treatment composition capable of imparting a good feel to the touch.

### Example 7

### Hair cream composition:

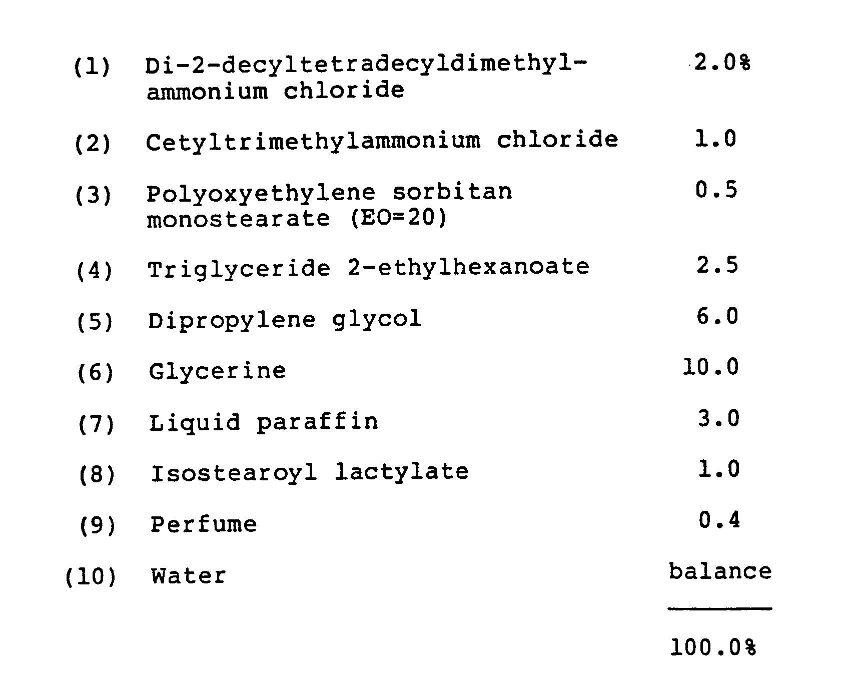

### (Preparation)

A uniform hot solution of (1), (2), (3), (4), (5), (6), (7) and (8) was added to hot (10) and cooled, to which (9) was added to obtain a hair cream composition capable of imparting a good feel to the touch.

### Example 8

### Styling lotion composition:

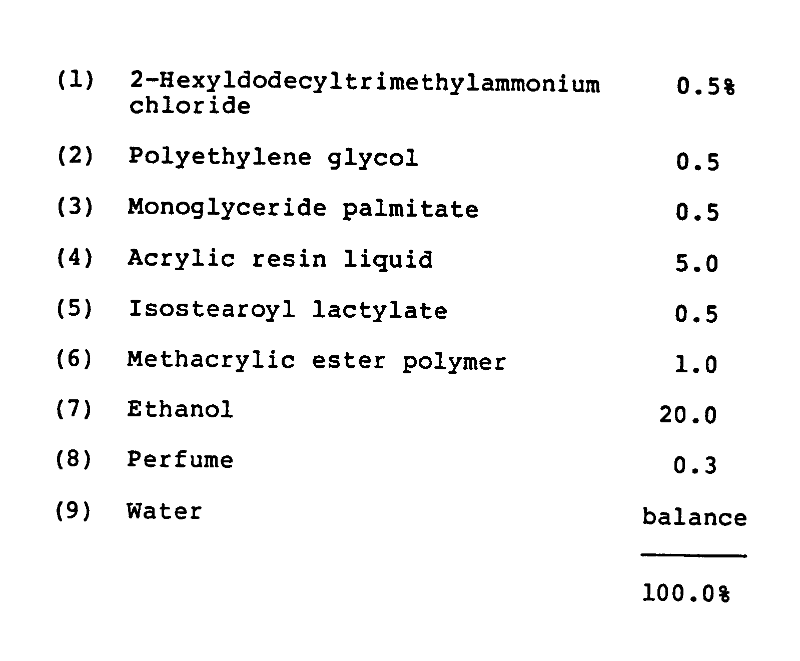

### (Preparation)

(1), (2), (3), (4), (5), (6) and (8) were added to (7) for uniform dispersion under agitation, to which (9) was added to obtain a setting composition which had a good feel to the touch and could impart a good hair style retention.

### Example 9

### Conditioning Mousse Composition:

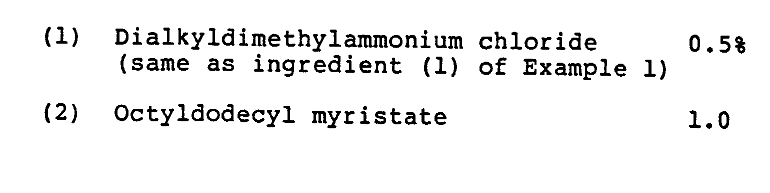

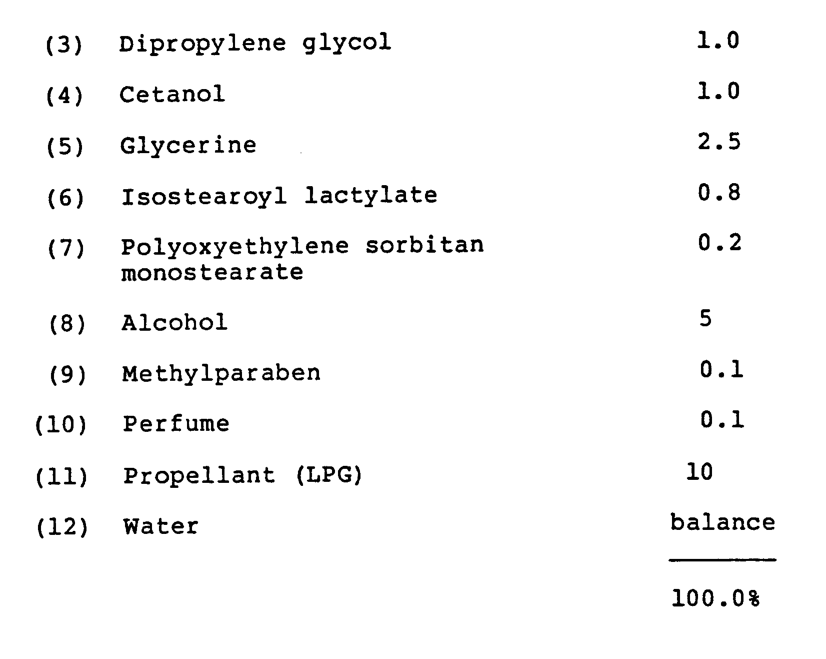

### (Preparation)

(9) was added to (12) and heated. A uniform heated solutin of (1), (2), (3), (4), (5), (6) and (7) was added to the mixture under agitation and cooled, to which (8) and (10) were added. The mixture was packed in an aerosol can, into which the propellant was also packed to obtain a mousse composition having a good feel to the touch.

### Example 10

Compositions having the formulations shown in Tables 7 and 8 were prepared to evaluate the stability upon storage and the rinsing properties.

### Preparation of the Compositions:

Among the ingredients (1) to (4) shown in Table 7, indicated were chosen and melted by heating to 70°C, and were added to water (5) heated to the same temperature. The mixture was agitated for emulsification, followed by cooling to room temperature while agitating, thereby obtaining hair rinse compositions.

### Results:

Shown in Tables 7 and 8.

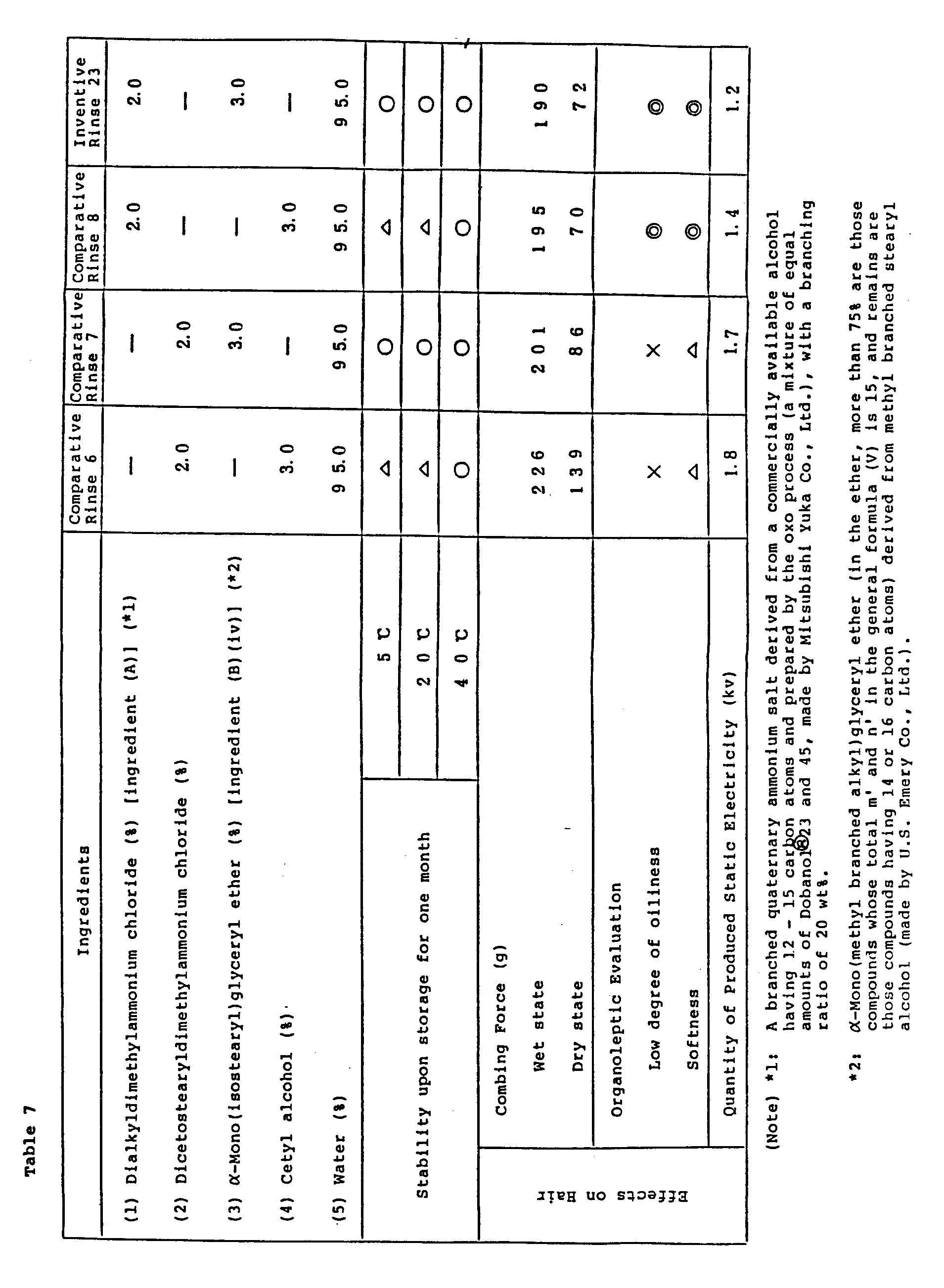

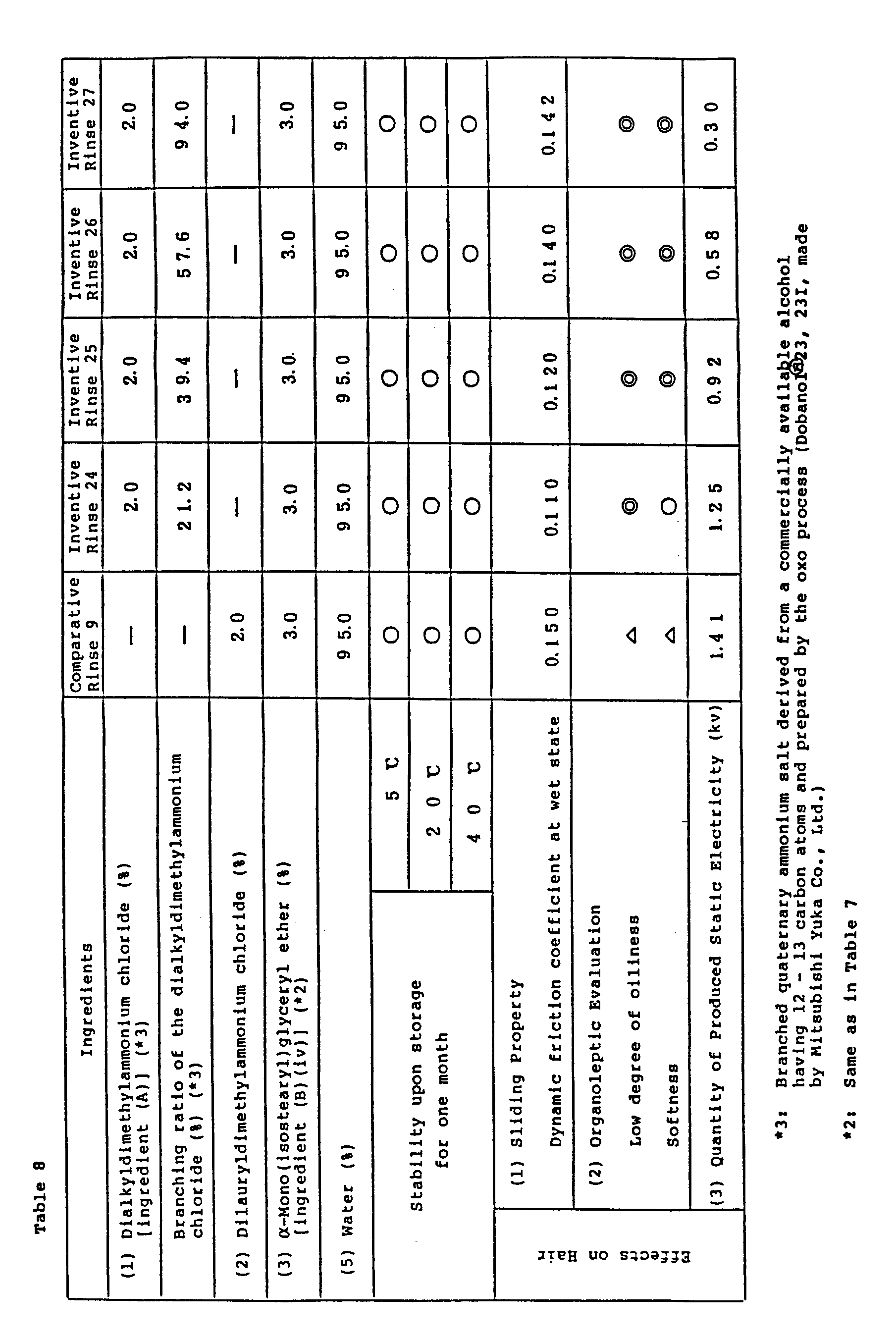

### Example 11

Compositions having the formulations shown in Table 9 were prepared to evaluate the stability upon storage at each temperature and the rinsing properties.

### Preparation of the Compositions:

The ingredients (1), (3) and (4) or (2), (3) and (4) melted by heating to 70°C were added to water (5) heated to the same temperature, and agitated for emulsification, followed by cooling to room temperature while agitating, thereby obtaining hair rinse compositions.

### Results:

Shown in Table 9.

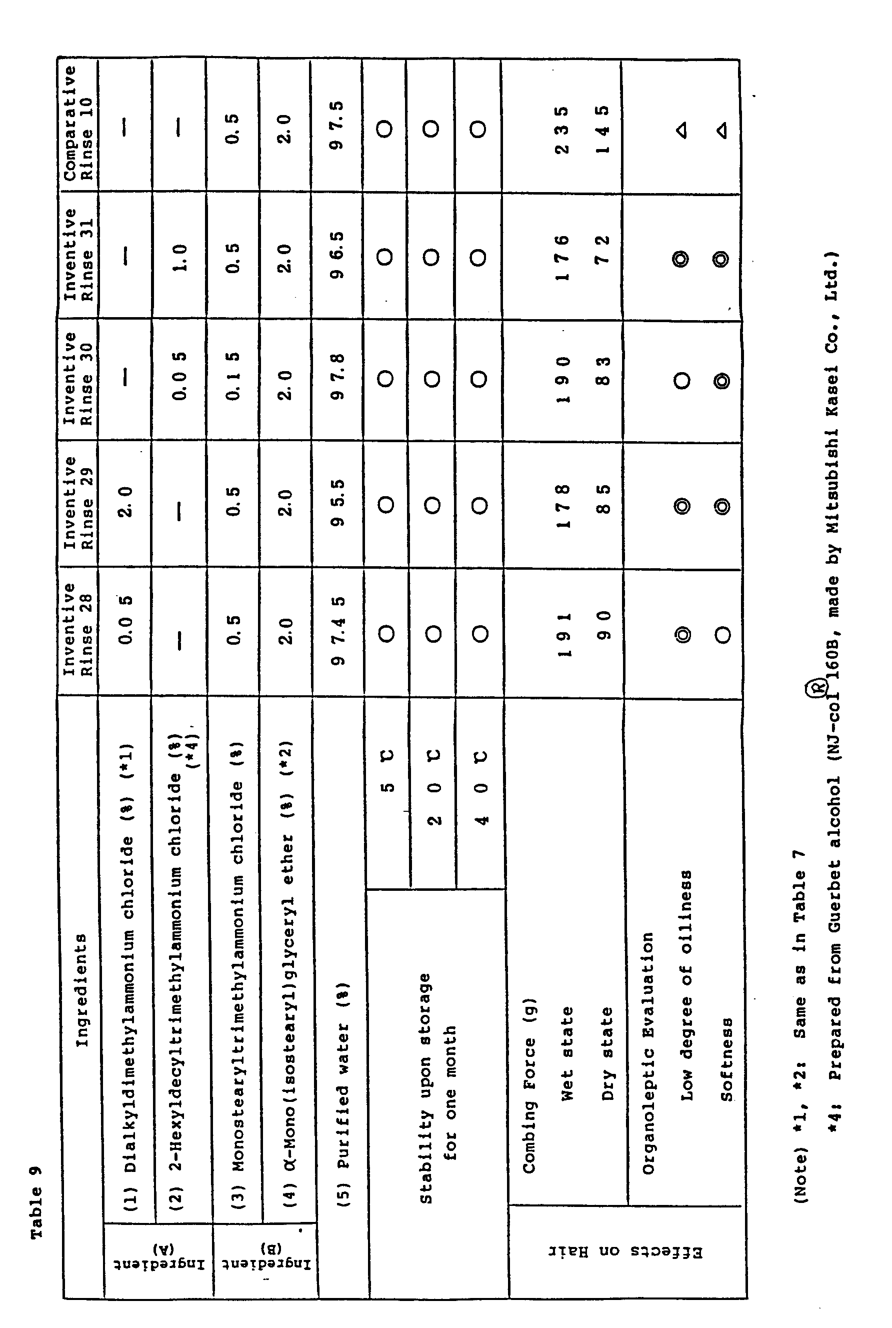

### Example 12

A hair rinse composition of the following formulation was prepared and its rinse property was evaluated by 19 female panelists using a paired comparison method. The comparison standards were determined as +2 when it was very good and as +1 when it was good. The results are shown in Table 10.

### Inventive Rinse 32:

| | |
|---|---|
| (1) Dialkyldimethylammonium chloride (*1) | 2.0% |
| (2) α-Mono(isostearyl)glyceryl ether (*2) | 3.0 |
| (3) Propylene glycol | 5.0 |
| (4) Water | 89.6 |
| (5) Perfume | 0.4 |

To (4) heated to 70°C was added a mixture of (1) - (3) which had been melted by heating at the same temperature as indicated above, which was agitated for emulsification, followed by cooling down to 45°C while agitating and further adding (5). The mixture was cooled down to room temperature under agitation to obtain a hair rinse composition. The marks "*1" and "*2" have the same meanings as described in Table 7.

### Comparative Rinse 11:

| | |
|---|---|
| (1) Dicetyldimethylammonium chloride | 2.0% |
| (2) α-Mono(isostearyl)glyceryl ether (*2) | 3.0 |
| (3) Propylene glycol | 5.0 |
| (4) Water | 89.6 |
| (5) Perfume | 0.4 |

To (4) heated to 70°C was added a mixture of (1) - (3) which had been melted by heating at the same temperature as indicated above and agitated for emulsification, which was cooled down to 45°C under agitation, followed by addition of (5) and cooling down to room temperature under agitation to obtain a hair rinse composition. The mark "*2" has the same meaning as described in Table 7.

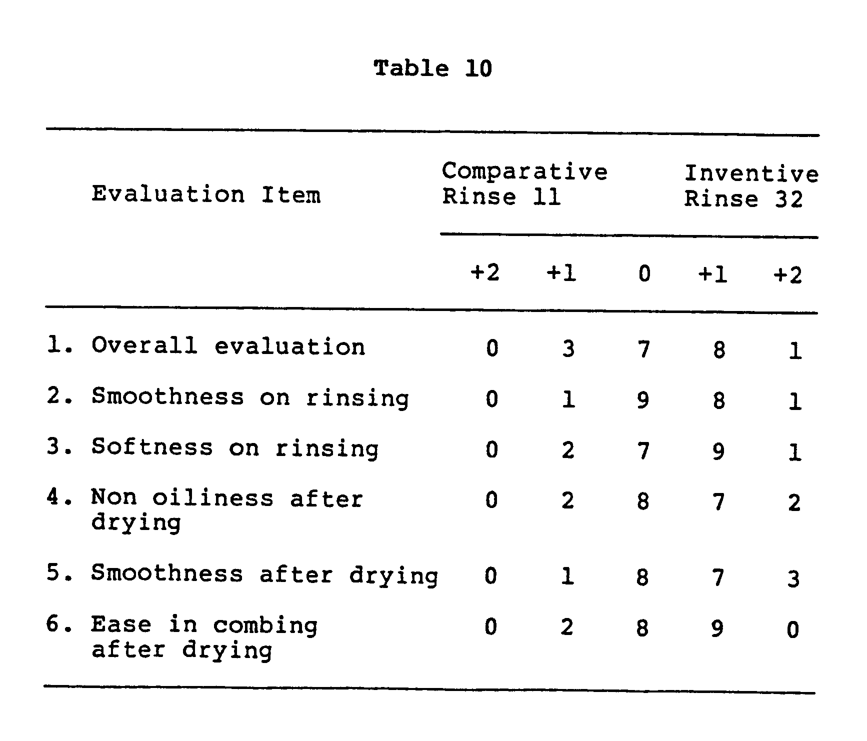

### Example 13

Compositions having the formulations shown in Table 11 were prepared to evaluate the stability upon storage at each temperature and the rinsing properties.

### Preparation of the Compositions:

The ingredients (1) to (8) melted by heating to 70°C were added to the ingredient (9) heated to the same temperature, and agitated for emulsification, followed by cooling to room temperature while agitating, thereby obtaining hair rinse composition. The results are shown in Table 11.

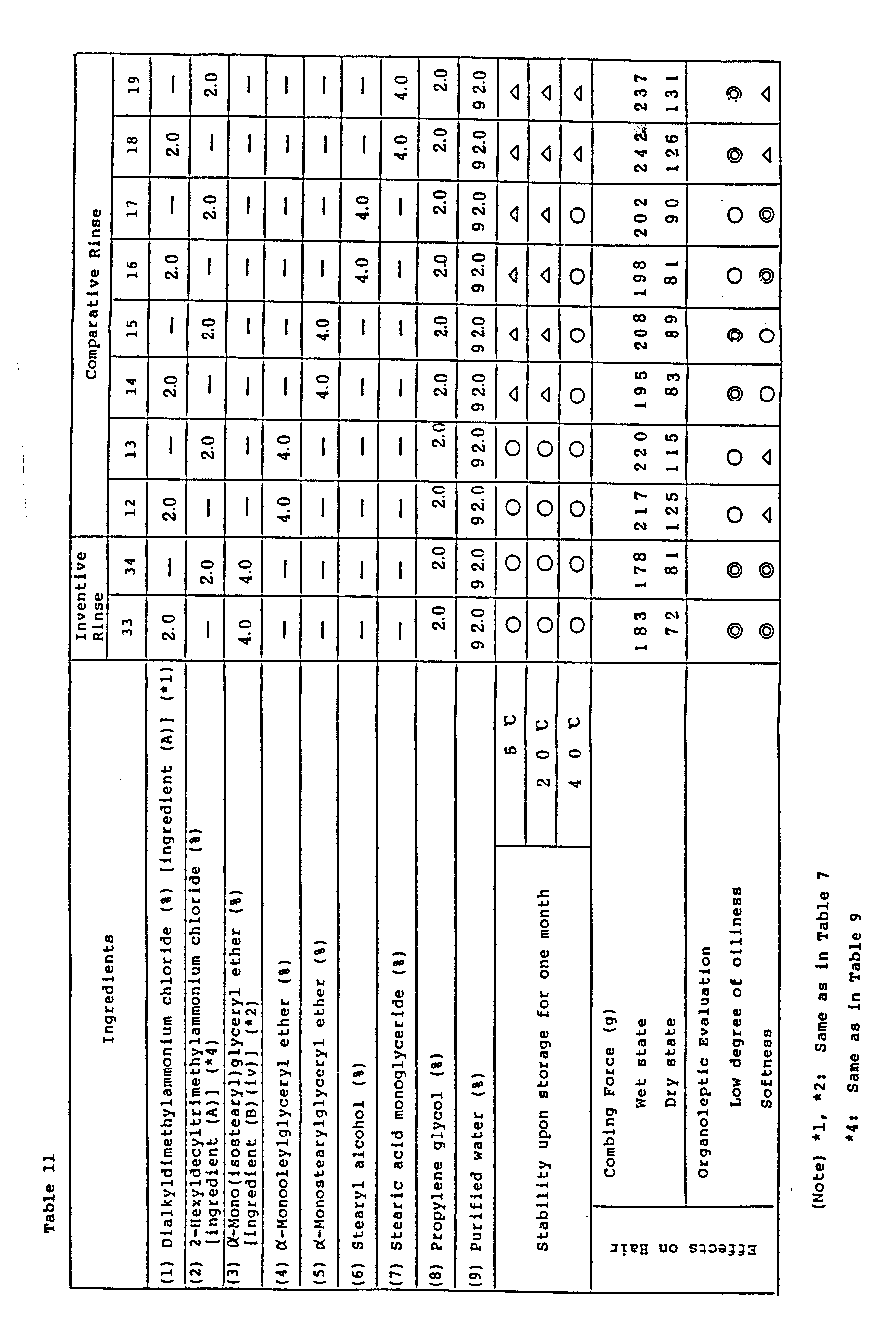

### Example 14

The ingredients (1) - (6) melted by heating to 70°C were added to a mixture of the ingredients (7), (8), (9) and (11) heated to the same temperature, and agitated for emulsification, followed by cooling to 45°C while agitating. Thereafter, the ingredient (10) was added to the above mixture and cooled down to room temperature under agitation to obtain hair rinse composition.

The thus obtained hair compositions showed good rinsing properties and a high stability upon storage at low temparature for a long period.

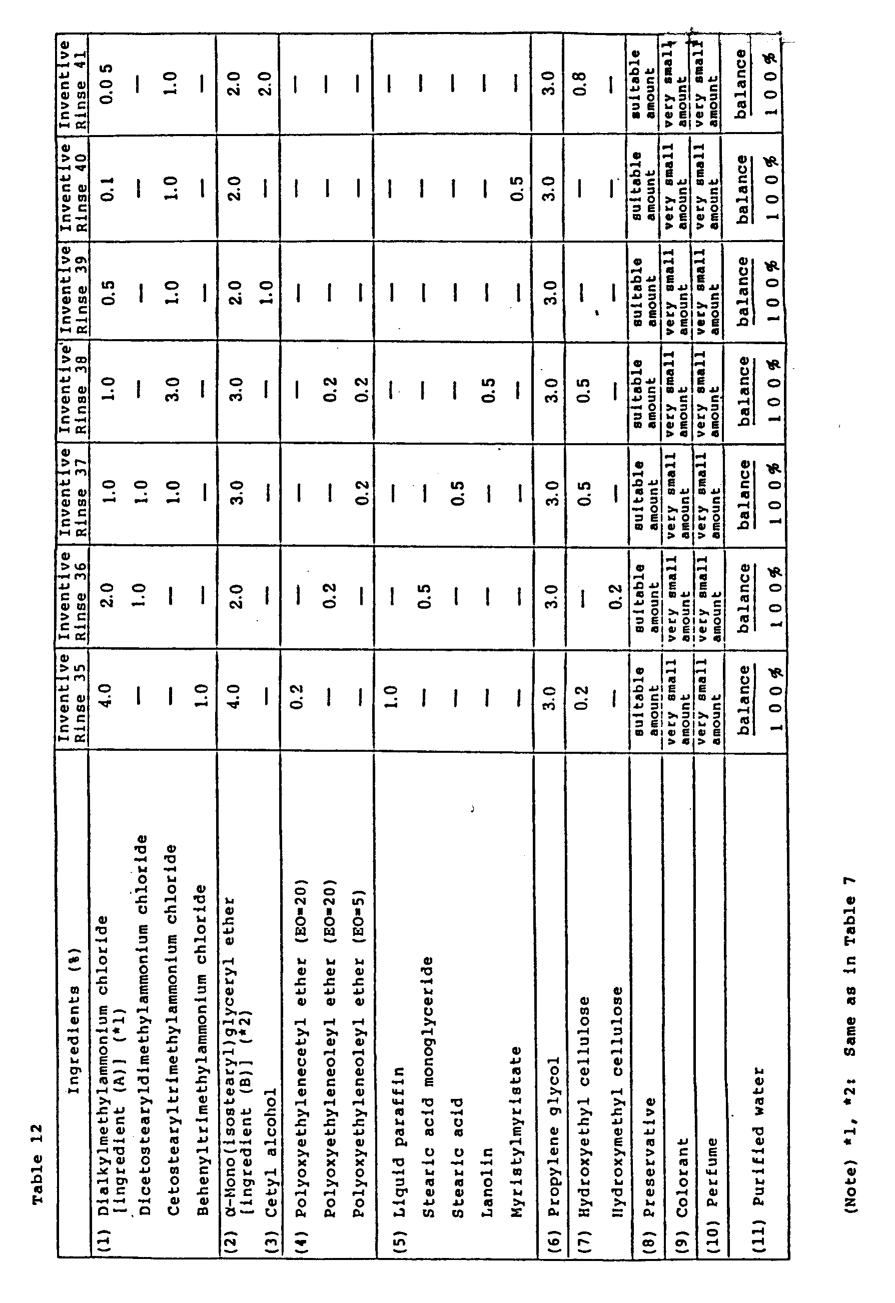

### Example 15

Hair rinse compositions were prepared in the same manner as in Example 11 to evaluate their properties. The results are shown in Table 13. In the table, "*2" has the same meaning as in Table 7.

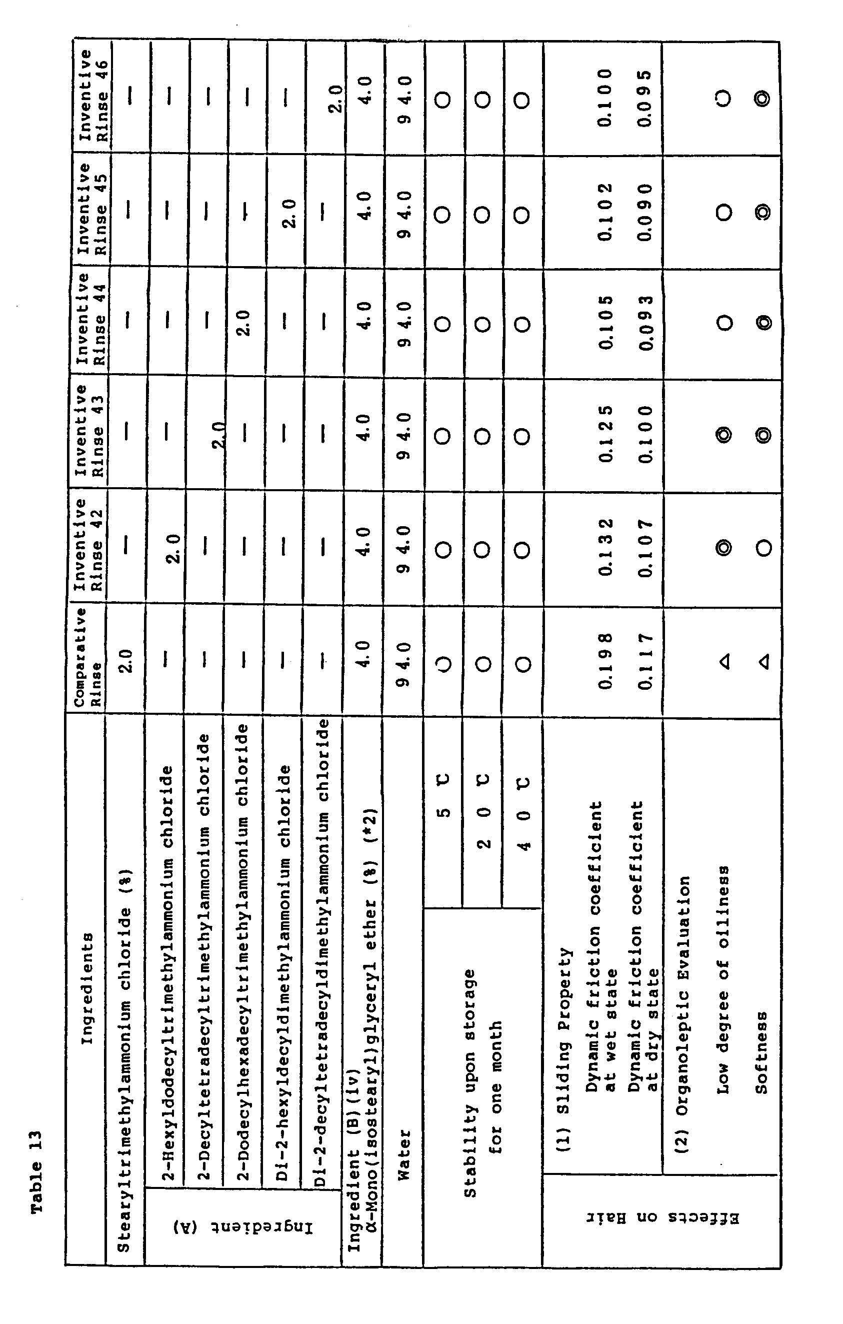

### Example 16

### Hair Treatment Composition

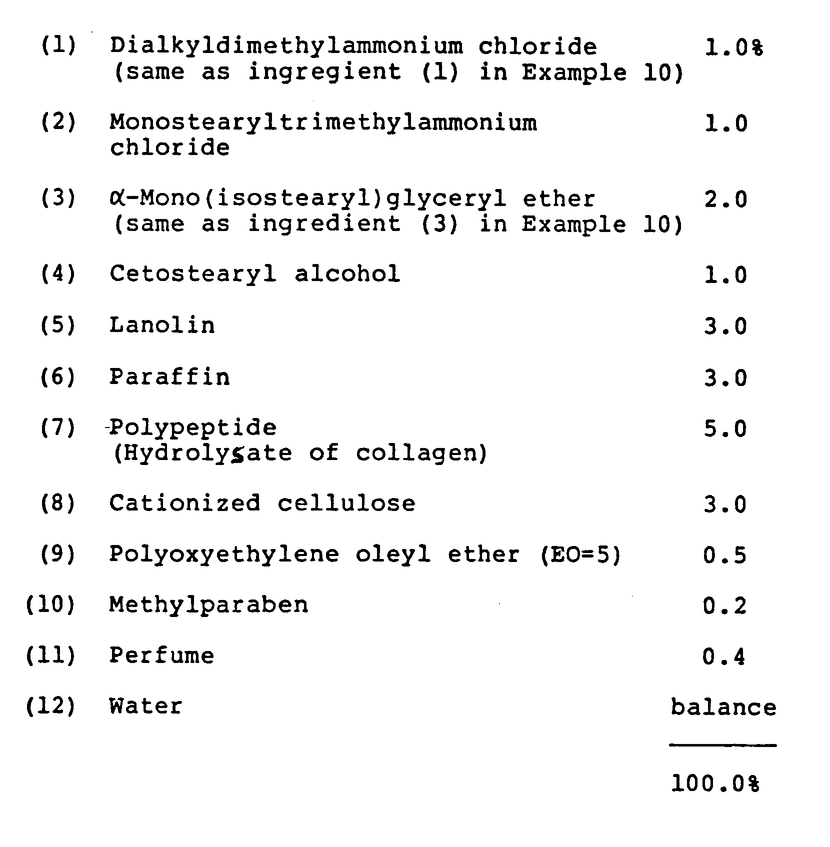

### (Preparation)

(7), (8) and (10) were dispersed in (12) and heated. A uniform solution of (1), (2), (3), (4), (5), (6) and (9) which had been heated under agitation was added to the dispersion and cooled, to which (11) was added to obtain a hair treatment composition having a high stability upon storage at low temperature and capable of imparting a good feel to the touch.

### Example 17

### Hair Cream Composition

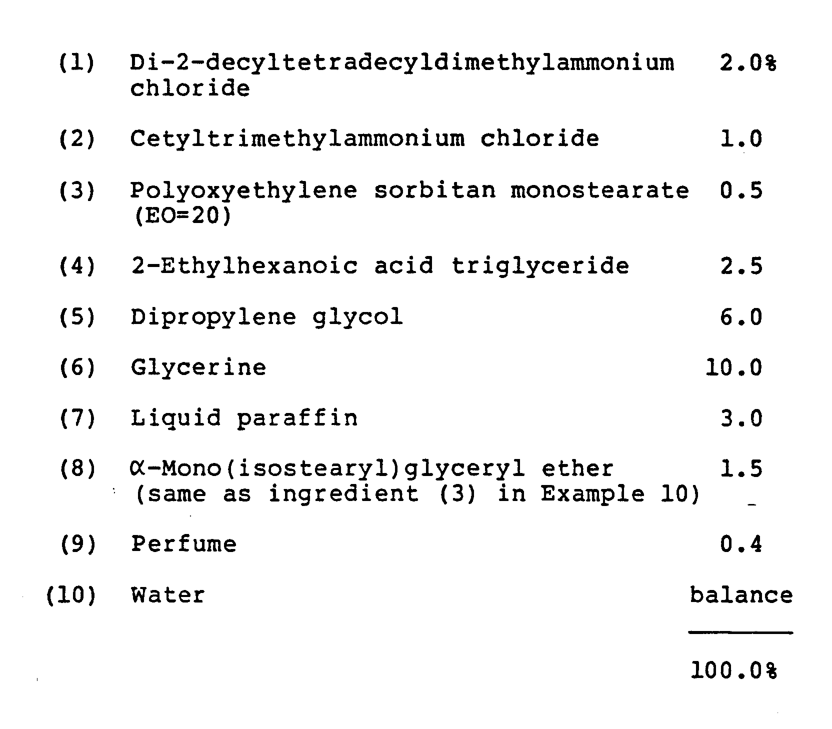

### (Preparation)

A uniform hot solution of (1), (2), (3), (4), (5), (6), (7) and (8) was added to hot (10) and cooled, to which (9) was added to obtain a hair cream composition having a high stability upon storage at low temperature and capable of imparting a good feel to the touch.

### Example 18

### Styling Lotion Composiiton

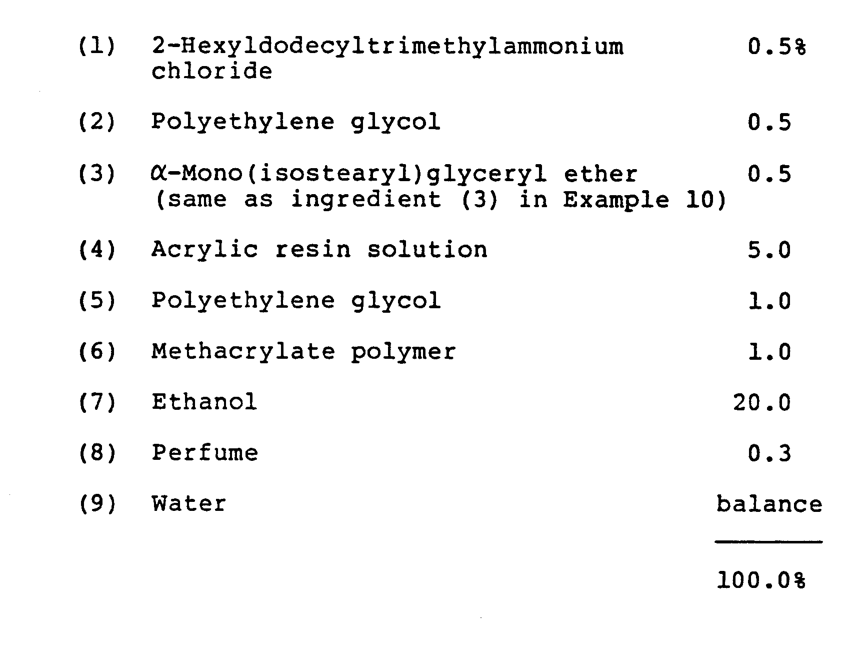

### (Preparation)

(1), (2), (3), (4), (5), (6) and (8) were added to (7) for uniform dispersion under agitation, to which (9) was added to obtain a setting composition having a high stability upon storage at low temperature and capable of imparting a good hair syle retention.

### Example 19

### Conditioning Mousse Composition

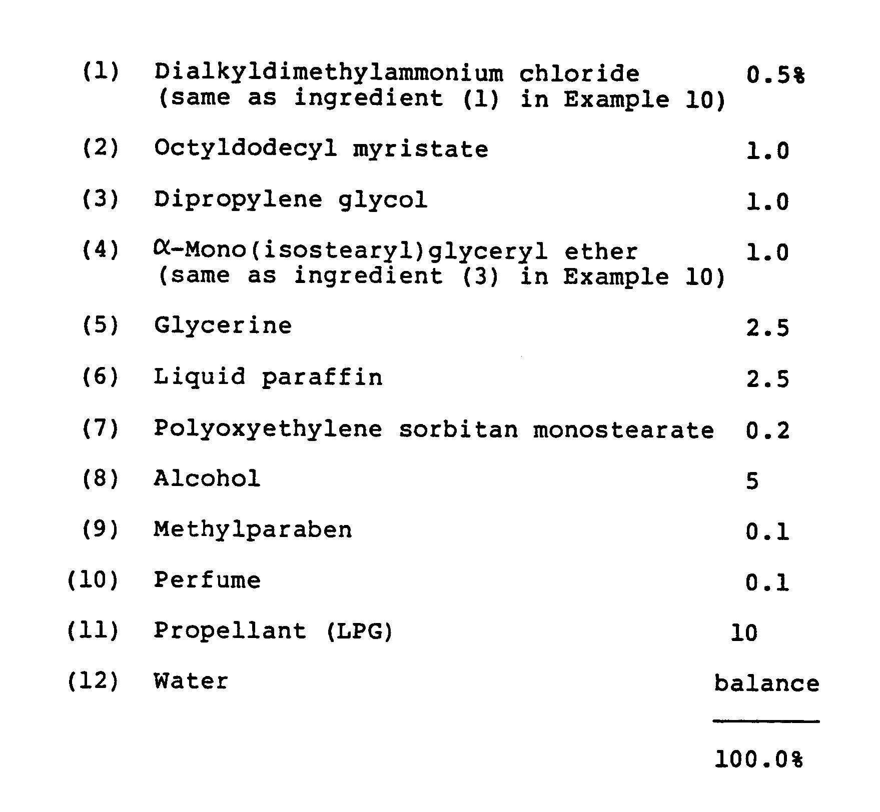

### (Preparation)

(9) was added to (12) and heated. A uniform heated solution of (1), (2), (3), (4), (5), (6) and (7) was added to the mixture under agitation and cooled, to which (8) and (10) were added. The mixture was packed in an aerosol can, into which the propellant was also packed to obtain a mousse composition having a high stability upon storage at low temperature and capable of imparting a good feel to the touch.

## Claims

1. A hair cosmetic composition comprising the following two ingredients (A) and (B):
(A) a total of 0.01 to 20 wt% of one or more of the following branched quaternary ammonium salts (i) or (ii): 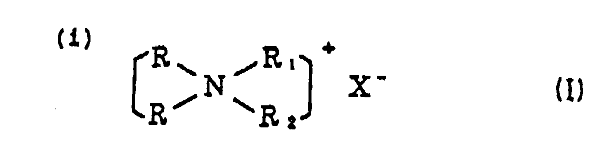 in which each R represents an alkyl group selected from the group consisting of (a) a branched alkyl group of the formula 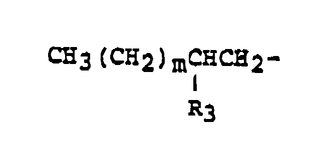 and (b) a linear alkyl group of the formula 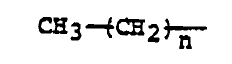 (in which R₃ represents a methyl or ethyl group, and m and n are independently a value determined such that, the total carbon atoms in the alkyl groups range from 8 to 16), and the branching ratio (a)/(a)+(b) is in the range of 10 to 100 wt%, R₁ and R₂ are independently a group selected from the group consisting of a benzyl group, and an alkyl group and a hydroxylalkyl group each having 1 to 3 carbon atoms, and X⁻ represents a halogen ion or an organic anion, and 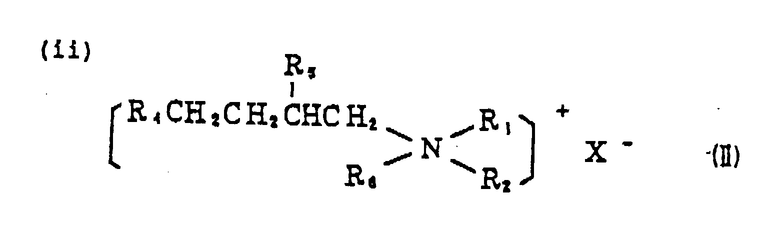 in which R₄ and R₅ independently represent an alkyl group having 2 to 16 carbon atoms, R₆ represents 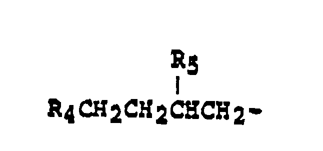 or an alkyl group having 1 to 3 carbon atoms, and R₁, R₂ and X⁻ have, respectively, the same meanings as in formula (I); and
(B) the following oily compound (iii) or (iv):
(iii) 0.1 to 5 wt% of a long-chain acyl compound of the following general formula (III) or its salt 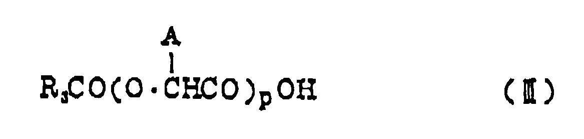 in which R₃CO represents an aliphatic linear or branched acyl group having 6 to 22 carbon atoms, A represents CH₃ or hydrogen, and p is an integer of 1 to 3; and
(iv) 0.1 to 30 wt% of α-mono(methyl branched alkyl)glyceryl ether of the following general formula (IV)
R'-OCH₂CH(OH)CH₂OH (IV)
in which R' represents a methyl branched alkyl group having 12 to 24 carbon atoms.

2. A hair cosmetic composition according to Claim 1, wherein R' in the formula (IV) of said α-mono(methyl branched alkyl)glyceryl ether of ingredient (B)-(iv) is a group represented by the following formula (V): 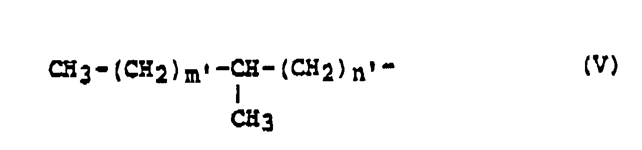 in which m' is an integer of 2 to 14, n' is an integer of 3 to 11, and the sum of m' and n' is 9 to 21.

## Revendications

1. Composition cosmétique capillaire comprenant les deux ingrédients (A) et (B) suivants :
(A) un total de 0,01 à 20% en poids d'au moins l'un des sels d'ammonium quaternaire ramifié (i) ou (ii) suivants: 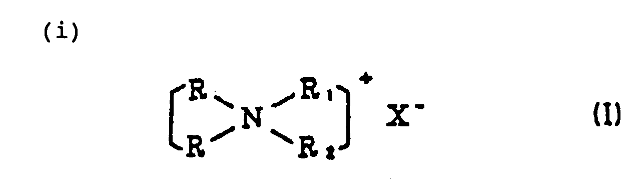 où :
- les R représentent chacun un groupe alkyle choisi dans le groupe constitué par (a) un groupe alkyle ramifié de la formule 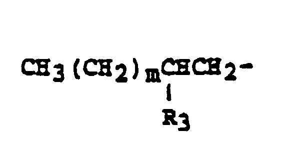 et (b) un groupe alkyle linéaire de la formule 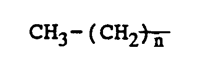 (dans lesquels R₃ représente un groupe méthyle ou éthyle, et m et n représentent indépendamment une valeur déterminée de telle sorte que le total des atomes de carbone dans les groupes alkyle se situe dans la plage de 8 à 16), et le rapport de ramification(a)/(a)+(b) se situe dans la plage de 10 à 100% en poids;
- R₁ et R₂ représentent indépendamment un groupe choisi dans le groupe constitué par un groupe benzyle, et un groupe alkyle et un groupe hydroxy alkyle ayant chacun 1 à 3 atomes de carbone ; et
- X⁻ représente un ion halogène ou un anion organique ; et 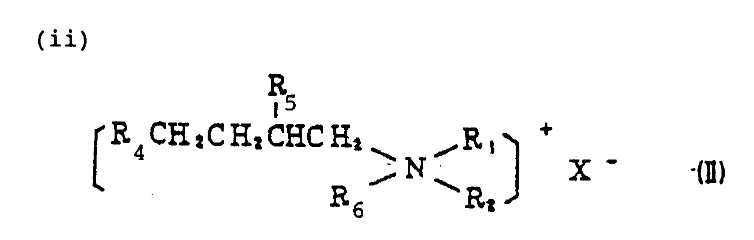
dans laquelle :
- R₄ et R₅ représentent indépendamment un groupe alkyle ayant 2 à 16 atomes de carbone ;
- R₆ représente 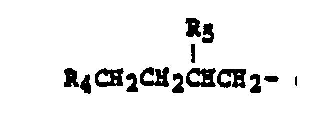
ou un groupe alkyle ayant 1 à 3 atomes de carbone ; et
- R₁, R₂ et X⁻ ont respectivement les mêmes significations que dans la formule (I) ; et
(B) le composé huileux (iii) ou (iv) suivant :
(iii) 0,1 à 5% en poids d'un composé acylé à longue chaîne de la formule générale (III) suivante, ou son sel : 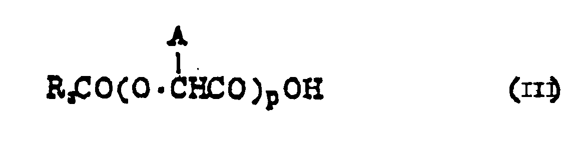 dans laquelle :
- R₃CO représente un groupe acyle aliphatique linéaire ou ramifié ayant 6 à 22 atomes de carbone ;
- A représente CH₃ ou hydrogène ; et
- p est un nombre entier de 1 à 3 ; et
(iv) 0,1 à 30% en poids d'un α-mono(alkyl à ramification méthyle) glycéryl éther de la formule générale (IV) suivante :
R'-OCH₂CH(OH)CH₂OH (IV)
dans laquelle R' représente un groupe alkyle à ramification méthyle ayant 12 à 24 atomes de carbone.

2. Composition cosmétique capillaire selon la revendication 1, dans laquelle R' dans la formule (IV) dudit α-mono(alkyle à ramification méthyle) glycéryl éther de l'ingrédient (B)-(iv) est un groupe représenté par la formule (V) suivante : 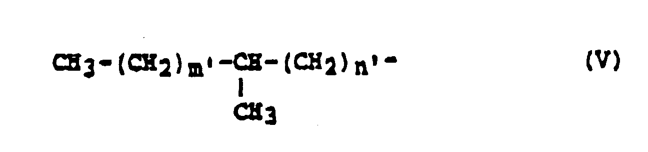 dans laquelle :
- m' est un nombre entier de 2 à 14 ;
- n' est un nombre entier de 3 à 11 ; et
- la somme de m' et n' est de 9 à 21.

## Patentansprüche

1. Eine Haarkosmetikzusammensetzung, die folgende Bestandteile (A) und (B) umfaßt:
(A) eine Gesamtmenge von 0,01 bis 20 Gew`-% von einem oder von mehreren der folgenden verzweigten quaternären Ammoniumsalze (i) oder (ii): 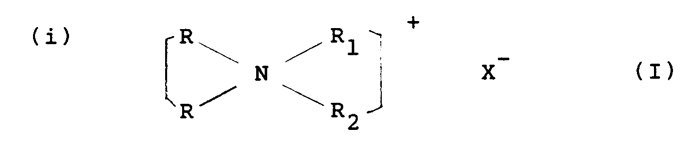 wobei jedes R für eine Alkylgruppe steht, die aus der Gruppe ausgewählt wird, bestehend aus (a) einer verzweigten Alkylgruppe der Formel 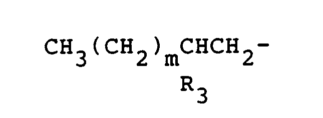 und
(b) einer linearen Alkylgruppe der Formel 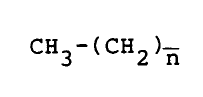 (in welcher R₃ für eine Methyl- oder Ethylgruppe steht, und m und n unabhängig voneinander einen solchen Wert haben, daß die Gesamtanzahl der Kohlenstoffatome in den Alkylgruppen in dem Bereich von 8 bis 16 liegt) und das Verzweigungsverhältnis (a)/(a)+(b) im Bereich von 10 bis 100 Gew.-% ist, R₁ und R₂ unabhängig voneinander einen Rest bedeuten, ausgewählt aus der Gruppe, die aus einer Benzylgruppe, einer Alkyl- und Hydroxyalkylgruppe mit 1 bis 3 Kohlenstoffatomen besteht, und X⁻ ein Halogenion oder ein organisches Anion repräsentiert, und 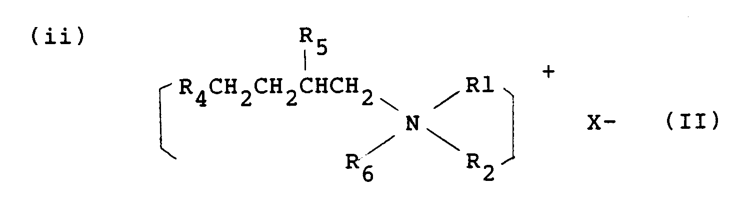 wobei R₄ und R₅ unabhängig für eine Alkylgruppe mit 2 bis 16 Kohlenstoffatomen stehen, R₆ für 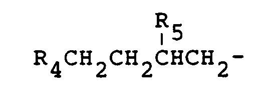 oder eine Alkylgruppe mit 1 bis 3 Kohlenstoffatomen steht und R₁, R₂ und X⁻ jeweils die gleichen Bedeutungen wie in Formel (I) haben; und
(B) die folgende ölige Verbindung (iii) oder (iv):
(iii) 0,1 bis 5 Gew.-% von einer langkettigen Acylverbindung der folgenden allgemeinen Formel (III) oder seinem Salz 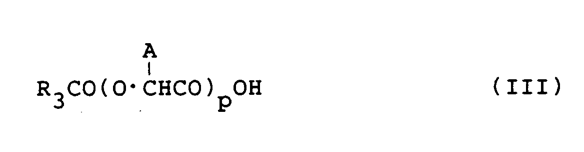 in welcher R₃CO eine aliphatische, lineare oder verzweigte Acylgruppe mit 6 bis 22 Kohlenstoffatomen repräsentiert, A für CH₃ oder Wasserstoff steht und p eine ganze Zahl von 1 bis 3 ist; und
(iv) 0,1 bis 30 Gew.-% eines α-Mono(methylverzweigt.-alkyl)glyceryl-ethers der folgenden allgemeinen Formel (IV)
R'-OCH₂CH(OH)CH₂OH (IV)
in welcher R' eine methylverzweigte Alkylgruppe mit 12 bis 24 Kohlenstoffatomen repräsentiert.

2. Eine Haarkosmetikzusammensetzung gemäß Patentanspruch 1, in der R' in der Formel (IV) des genannten α-Mono(methylverzweigt.-alkyl)glyceryl-ethers des Bestandteils (B)-(iv) eine Gruppe ist, die durch die folgende Formel (V) dargestellt wird: 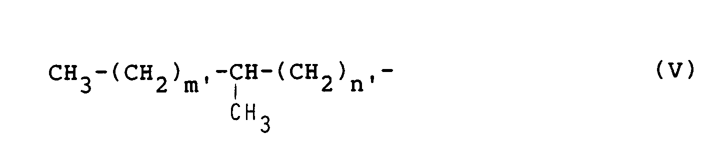 in welcher m' eine ganze Zahl von 2 bis 14, n' eine ganze Zahl von 3 bis 11 und die Summe von m' und n' 9 bis 21 ist.
